# EUROPÄISCHE PATENTSCHRIFT

(11) **EP 2 164 453 B1**
(45) Veröffentlichungstag und Bekanntmachung des Hinweises auf die Patenterteilung: **18.07.2018**
(21) Anmeldenummer: 08773395.2
(22) Anmeldetag: 12.06.2008
(51) Int. Cl.: A61K 8/31, A61Q 19/00, A61Q 15/00, A61Q 17/04, A61Q 19/04, A61K 31/01

(54) **KOSMETISCHE ZUBEREITUNGEN ENTHALTEND KOHLENWASSERSTOFFE**
COSMETIC PREPARATIONS CONTAINING HYDROCARBONS
PRÉPARATIONS COSMÉTIQUES CONTENANT DES HYDROCARBURES

(30) Priorität: 19.06.2007 EP 07011967; 20.06.2007 EP 07075513; 04.03.2008 DE 102008012458; 03.04.2008 DE 102008017032; 03.04.2008 DE 102008017034; 07.05.2008 DE 102008022433
(43) Veröffentlichungstag der Anmeldung: 24.03.2010
(73) Patentinhaber: Cognis IP Management GmbH, 40589 Dusseldorf (DE)
(72) Erfinder: DIERKER, Markus, 40593 Düsseldorf (DE); RICHTER, Joachim, 40227 Düsseldorf (DE)
(74) Vertreter: BASF IP Association
(86) Internationale Anmeldenummer: PCT/EP2008/004703
(87) Internationale Veröffentlichungsnummer: WO 2008/155061

(56) Entgegenhaltungen:
- EP-A1- 2 014 274
- WO-A1-00/02992
- WO-A1-98/58624
- WO-A1-2004/011581
- WO-A1-2007/068371
- WO-A2-2008/155057
- WO-A2-2009/080966

## Beschreibung

Die vorliegende Erfindung betrifft kosmetische und/oder pharmazeutische Zubereitungen, enthaltend Kohlenwasserstoffe sowie die Verwendung von Kohlenwasserstoffen als Ölkörper und/oder Dispergiermittel.

### Stand der Technik

Sensorisch leichte Ölkörper, so genannte "light emollients", werden von der kosmetischen Industrie in einer Vielzahl von Formulierungen verwendet. Insbesondere für die dekorative Kosmetik bzw. in pflegenden Formulierungen werden so genannte "leichte" Komponenten eingesetzt. Bei diesen Komponenten kann es sich beispielsweise um flüchtige, cyclische Silikone (z.B. Cyclopentasiloxan oder Cyclomethicone) oder Kohlenwasserstoffe aus petrochemischen Prozessen handeln. Bei den zuletzt genannten Stoffen handelt es sich aufgrund ihrer Herstellung überwiegend um Gemische aus linearen, cyclischen und verzweigten Kohlenwasserstoffen, deren Flammpunkt durchaus unter 50 °C (wie z.B. beim Isododecan) liegen kann. Beispiele und anwendungstechnische Beschreibungen derartiger Formulierungen können in Standardwerken, wie zum Beispiel: 'Handbook of Cosmetic Science and Technology', A. Barel, M. Paye, H. Maibach, Marcel Dekker Inc. 2001 nachgelesen werden. Aus toxikologischen, ökologischen bzw. sicherheitstechnischen Gründen besteht jedoch in Zukunft Bedarf nach alternativen Rohstoffen für derartige Formulierungsaufgaben.

Die Aufgabe der Erfindung bestand darin, alternative Rohstoffe zu finden, die ökologisch bzw. toxikologisch unbedenklich sind. Dabei war es insbesondere von Interesse Rohstoffe bereit zu stellen, welche ohne aufwendige Reinigungsschritte direkt in kosmetischen bzw. pharmazeutischen Zubereitungen eingesetzt werden können. Vorzugsweise sollten diese Rohstoffe auf Basis nachwachsender Rohstoffe erhältlich sein. Diese Rohstoffe sollten in typischen kosmetischen und/oder pharmazeutischen Formulierungen ohne anwendungstechnische Einschränkungen direkt eingesetzt werden können. Darüber hinaus sollten die Rohstoffe gegenüber den Kohlenwasserstoff Gemischen des Standes der Technik eine verbesserte Sensorik aufweisen, wünschenswert war auch, dass diese Rohstoffe eine bessere Hautverträglichheit aufweisen. Von besonderem Interesse war es, Rohstoffe bereit zu stellen, welche hinsichtlich ihrer sensorischen Einsatzmöglichkeiten mit Silikonölen, insbesondere mit niedrigviskosen Silikonölen, wie z.B. Dimethiconen vergleichbar sind. Wünschenswert war es insbesondere Rohstoffe zu Verfügung zu stellen, welche sich als Ersatzstoffe für Silikonöle eignen. Darüber hinaus war es von Interesse Rohstoffe bereit zu stellen, die gegenüber den Rohstoffen des Standes der Technik eine verbesserte CO₂-Bilanz aufweisen.

Eine weitere Aufgabe bestand darin Rohstoffe zur Verfügung zu stellen, welche eine stabile Formulierung mit AP/Deo (=Antiperspirant/Desodorant) Wirkstoffen ermöglicht. Kosmetische Zubereitungen der Kategorie Antiperspirantien/Desodorantien, insbesondere in so genannten "Stick-Formulierungen" haben immer noch das Problem der unzureichenden Stabilität der kosmetischen Grundlage, insoweit daß sich geruchliche Veränderungen während der Lagerung ergeben. Eine weitere Aufgabe der Erfindung bestand daher darin, Rohstoffe zu Verfügung zu stellen, welche es ermöglichen antiperspirierende bzw. desodorierende Zubereitungen, insbesondere solche in "Stick-Formulierung" stabil bereit zu stellen. Diese Zubereitungen sollten insbesondere bei längerer Lagerung keine unerwünschten Geruchsentwicklungen zeigen. Eine weitere Aufgabe bestand darin Rohstoffe zu Verfügung zu stellen, welche einen sensorisch "leichten" Eindruck vermitteln, möglichst bei gleichzeigter verbesserter Hautverträglichkeit, insbesondere in Kombination mit UV-Lichtschutzfiltern sowie in Verbindung mit Selbstbräunern. Von besonderem Interesse ist die Bereitstellung von neuen Rohstoffen, die in Formulierungen der dekorativen Kosmetik einen sensorisch vorteilhaften Eindruck ermöglichen. An Formulierungen der dekorativen Kosmetik, wie beispielsweise Lippenstifte, Lidschatten, Mascara, Nagellack etc. werden aufgrund des Applikationsorts (hauptsächlich Gesicht und Hände) erhöhte Anforderungen an die Sensorik, insbesonderen die Flüchtigkeit gestellt, damit diese Produkte nicht den Eindruck von "Schwere" vermitteln. Desweiteren ist bei diesen Produkten eine gute Dispergierbarkeit von Pigmenten wünschenswert.

WO2004/011581 A1 beschreibt das Lanolin, welches ebenfalls Anteile an Kohlenwasserstoffen mit ungeradzahligen Kohlenstoffanzahlen enthält. WO2007/068371 A1 vermittelt einen Zugang zu Gemischen ungeradzahliger Kohlenwasserstoffe über die reduktive Dehydroxymethylierung primärer Alkohole.

### Beschreibung der Erfindung

Ein Gegenstand der Erfindung betrifft kosmetische und/oder pharmazeutische Zubereitungen, enthaltend **0,1 bis 80 Gew.% eines** linearen **Kohlenwasserstoffs,** ausgewählt aus der Gruppe bestehend aus C15, C17 und C19 Kohlenwasserstoffen oder Mischungen davon, wobei diese Kohlenwasserstoffe mehr als 95 Gew.-%, bezogen auf die Summe der in der Zubereitung enthaltenen Kohlenwasserstoffe, ausmachen. Die Gew.-% beziehen sich auf das Gesamtgewicht der kosmetischen und/oder pharmazeutischen Zubereitung.

Als Kohlenwasserstoffe werden organische Verbindungen bezeichnet, die nur aus Kohlenstoff und Wasserstoff bestehen. Sie umfassen sowohl cyclische als auch acyclische (=aliphatische) Verbindungen. Sie umfassen sowohl gesättigte wie einfach oder mehrfach ungesättigte Verbindungen. Die Kohlenwasserstoffe können linear oder verzweigt sein. Je nach Anzahl der Kohlenstoffatome im Kohlenwasserstoff kann man die Kohlenwasserstoffe einteilen in ungradzahlige Kohlenwasserstoffe (wie beispielsweise Nonan, Undecan, Tridecan) oder geradzahlige Kohlenwasserstoffe (wie beispielsweise Octan, Dodecan, Tetradecan). Je nach Art der Verweigung kann man die Kohlenwasserstoffe einteilen in lineare (= unverzweigte) oder verzweigte Kohlenwasserstoffe. Gesättigte, aliphatische Kohlenwasserstoffe werden auch als Paraffine bezeichnet.

Der Begriff "CX-Kohlenwasserstoff" umfasst Kohlenwasserstoffe mit einer C-Zahl von X (wobei X eine ganze Zahl darstellt), so umfasst beispielsweise der Begriff C11-Kohlenwasserstoff alle Kohlenwasserstoffe mit einer C-Zahl von 11. Der Begriff "Kohlenstoff Zahl" oder "C-Zahl" umfasst alle im Kohlenwasserstoff vorhandenen C-Atome. Er beträgt somit z.B. für Undecan = 11 oder für Tridecan = 13.

Die Erfindung umfasst sowohl kosmetische und/oder pharmazeutische Zubereitungen, die nur einen der genannten Kohlenwasserstoffe enhält als auch beliebige Mischungen der genannten Kohlenwasserstoffe.

In einer bevorzugten Ausführungsform der Erfindung enthalten die kosmetischen und/oder pharmazeutischen Zubereitungen gesättige Kohlenwasserstoffe, ausgewählt aus der Gruppe bestehend aus C15, C17 und C19 Kohlenwasserstoffe.

In einer bevorzugten Ausführungsform der Erfindung enthalten die kosmetischen und/oder pharmazeutischen Zubereitungen gesättige, lineare Kohlenwasserstoffe, ausgewählt aus der Gruppe bestehend aus C15, C17 und C19 Kohlenwasserstoffe.

In einer bevorzugten Ausführungsform der Erfindung enthalten die kosmetischen und/oder pharmazeutischen Zubereitungen, Kohlenwasserstoffe, ausgewählt aus der Gruppe bestehend aus n-Pentadecan, n-Heptadecan und n-Nonadecan. Folgende Tabelle führt wichtige physikochemische Eigenschaften der bevorzugt zu verwendenden Kohlenwasserstoffe auf:

| | **Nonan** | | **Undecan** | | **Tridecan** | | **Pentadecan** | |
|---|---|---|---|---|---|---|---|---|
| Summenformel | C₉H₂₀ | | C₁₁H₂₄ | | C₁₃H₂₈ | | C₁₅H₃₂ | |
| CAS-Nummer | [111-84-2] | | [1120-21-4] | | [629-50-5] | | [629-62-9] | |
| EG-Nummer | [203-913-4] | | [214-300-6] | | [211-093-4] | | [211-098-1] | |
| Molmasse (g/mol) | 128,26 | | 156,31 | | 184,37 | | 212,42 | |
| Schmelzpunkt (°C) | -51 | | -26 | | -5 | | 9,9 | |
| Siedepunkt (°C) | 151 | | 196 | | 234 | | 268-270 | |
| Dichte (g/ml) | 0,72 | | 0,74 | | | | 0,769 | |
| Viskosität (cP bei 20 °C) | 0,711 | | | | | | | |
| Flammpunkt (°C) | 31 | | 60 | | 102 | | 132 | |
| Zündtemperatur (°C) | 205 | | | | | | | |

| | **Heptadecan** | | **Nonadecan** | | **Henicosan** | | **Tricosan** | |
|---|---|---|---|---|---|---|---|---|
| Summenformel | C₁₇H₃₆ | | C₁₉H₄₀ | | C₂₁H₄₄ | | C₂₃H₄₈ | |
| CAS-Nummer | [629-78-7] | | [629-92-5] | | [629-94-7] | | [638-67-5] | |
| Molmasse (g/mol) | 240,47 | | 268,53 | | 296,58 | | 324,63 | |
| Schmelzpunkt (°C) | 21 | | 32-34 | | 40,5 | | 48-50 | |
| Siedepunkt (°C) | 302 | | 330 | | 356,5 | | 380 | |
| Dichte (g/ml) | 0,777 | | | | 0,792 | | 0,797 | |
| Flammpunkt (°C) | 148 | | 168 | | | | | |

Die kosmetischen und/oder pharmazeutischen Zubereitungen können weitere Kohlenwasserstoffe, wie beispielsweise Paraffine enthalten. Dabei enthalten die kosmetischen und/oder pharmazeutischen Zubereitungen mehr als 95 Gew.-% Kohlenwasserstoffe, ausgewählt aus der Gruppe bestehend aus C15, C17 und C19 Kohlenwasserstoffe, bezogen auf die Summe der in der Zubereitung enthaltenen Kohlenwasserstoffe. Die Bezugsgröße "Summe der Kohlenwasserstoffe" umfasst alle in der kosmetischen und/oder pharmazeutischen Zubereitung enthaltenen Kohlenwasserstoffe, unabhängig von ihrer Kohlenstoffzahl.

Ein weiterer Gegenstand der Erfindung betrifft die Verwendung von linearen Kohlenwasserstoffen, ausgewählt aus der Gruppe bestehend aus C15, C17 und C19 Kohlenwasserstoffen oder Mischungen davon, wobei diese Kohlenwasserstoffe mehr als 95 Gew.-%, bezogen auf die Summe der in der Mischung enthaltenen Kohlenwasserstoffe, ausmachen als Ölkörper und/oder Dispergiermittel in kosmetischen und/oder pharmazeutischen Zubereitungen. Ein weiterer Gegenstand der Erfindung betrifft die Verwendung derartiger Kohlenwasserstoff(gemisch)e in kosmetischen Zubereitungen zur Pflege von Haut und/oder Haaren.

Ein weiterer Gegenstand der Erfindung betrifft die Verwendung derartiger Kohlenwasserstoff(gemisch)e in kosmetischen Zubereitungen zum Sonnenschutz.

Ein weiterer Gegenstand der Erfindung betrifft die Verwendung derartiger Kohlenwasserstoff(gemisch)e in Zubereitungen der dekorativen Kosmetik, wie beispielsweise Lippenstifte, Lip Gloss, Lidschatten, Wimperntuschen (Mascara), Lidstifte (Kajal), Nagellack sowie in Make-up Formulierungen jeder Art (Puder, Creme, Foundation, Abdeckstifte etc.).

Ein weiterer Gegenstand der Erfindung betrifft die Verwendung derartiger Kohlenwasserstoff(gemisch)e in Zubereitungen zur Reinigung von Haut und/oder Haaren, wie beispielsweise Shampoos, Duschgels, Badezusätze, Conditioner etc.

Ein weiterer Gegenstand der Erfindung betrifft die Verwendung derartiger Kohlenwasserstoff(gemisch)e zur Herstellung von feinteiligen Emulsionen, so z.B. Nanoemulsionen, Microemulsionen oder PIT Emulsionen. In solchen feinteiligen Emulsionen liegen die Öltröpfchen in der Regel im Bereich von 10 bis 1000 nm, vorzugsweise 100 bis 500 nm Durchmesser vor. Diese werden nach dem Fachmann bekannten Verfahren hergestellt, für PIT Emulsionen beispielsweise in Parfümerie und Kosmetik, 77. Jahrgang, Nr. 4/96, S. 250 - 254 von Wadle **et al.** beschrieben.

### Herstellung der Kohlenwasserstoffe

Die erfindungsgemäßen Kohlenwasserstoffe können beispielsweise durch reduktive Demethylierung nach dem Fachmann bekannten Methoden erhalten werden. Besonders geeignet zur Herstellung der erfindungsgemäßen Kohlenwasserstoffe ist das in der internationalen Anmeldung WO2007/068371 A1 (Cognis) beschrieben Verfahren der reduktiven Dehydroxymethylierung ausgehend von Fettalkoholen pflanzlichen Ursprungs. Dabei können beispielsweise C12 oder C14 Fettalkohole dem beschriebenen Verfahren unterzogen werden und die so erhaltenen C11 oder C13 Kohlenwasserstoffe zu den erfindungsgemäßen Zubereitungen gemischt werden.

Mit den erfindungsgemäßen Kohlenwasserstoffen werden leichte, stabile kosmetische und/oder pharmazeutische Zubereitungen erhalten, dies ist insbesondere dann der Fall, wenn die Kohlenwasserstoffe zusammen mit Antiperspirant-/Desodorant-Wirkstoffen eingesetzt werden.

Ein Gegenstand der Erfindung betrifft daher kosmetische und/oder pharmazeutische Zubereitungen, enthaltend **0,1 bis 80 Gew.% Kohlenwasserstoffe,** ausgewählt aus der Gruppe anspruchsgemäßer Kohlenwasserstoff(gemisch)e und mindestens einen Antiperspirant-/ Desodorant-Wirkstoff. Erfindungsgemäß sind als **Antiperspirant/Desodorant Wirkstoff** alle Wirkstoffe geeignet, die Körpergerüchen entgegen wirken, diese überdecken oder beseitigen. Körpergerüche entstehen durch die Einwirkung von Hautbakterien auf apokrinen Schweiß, wobei unangenehm riechende Abbauprodukte gebildet werden. Als Antiperspirant /Desodorant Wirkstoffe eignen sich insbesondere Verbindungen ausgewählt aus der Gruppe bestehend Antiperspirantien, Esteraseinhibitoren, bakterizide bzw. bakteriostatische Wirkstoffe und/oder schweißabsorbierende Substanzen.

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt. Die erfindungsgemäßen Zubereitungen können die Antiperspirantien in Mengen von 1 bis 50, vorzugsweise 5 bis 30 und insbesondere 8 bis 25 Gew.-% - bezogen auf das Gesamtgewicht der kosmetischen und/oder pharmazeutischen Zubereitung - enthalten.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen® CAT, Cognis GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäure-diethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Zubereitungen können die Esteraseinhibitoren in Mengen von 0,01 bis 20, vorzugsweise 0,1 bis 10 und insbesondere 0,3 bis 5 Gew.-% - bezogen auf das Gesamtgewicht der kosmetischen und/oder pharmazeutischen Zubereitung - enthalten.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Die erfindungsgemäßen Zubereitungen können die bakterizide bzw. bakteriostatische Wirkstoffe in Mengen von 0,01 bis 5 und vorzugsweise 0,1 bis 2 Gew.-% - bezogen auf das Gesamtgewicht der kosmetischen und/oder pharmazeutischen Zubereitung - enthalten.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 30, vorzugsweise 1 bis 20 und insbesondere 2 bis 8 Gew.-% - bezogen auf das Gesamtgewicht der kosmetischen und/oder pharmazeutischen Zubereitung - enthalten.

Mit den erfindungsgemäßen Kohlenwasserstoffen werden sensorisch leichte, kosmetische und/oder pharmazeutische Zubereitungen erhalten, dies ist insbesondere dann der Fall, wenn die Kohlenwasserstoffe zusammen mit UV-Lichtschutzfiltern eingesetzt werden.

Ein Gegenstand der Erfindung betrifft daher kosmetische und/oder pharmazeutische Zubereitungen, enthaltend **0,1 bis 80 Gew.% Kohlenwasserstoffe,** ausgewählt aus der Gruppe anspruchsgemäßer Kohlenwasserstoff(gemisch)e und mindestens einen **UV-Lichtschutzfilter.**

Erfindungsgemäß sind als **UV-Lichtschutzfilter** bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
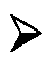 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
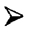 3-(4'-Trimethylammonium) benzyliden- bornan-2-on-methylsulfat (Mexoryl SO)
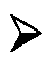 3,3'-(1,4-Phenylendimethin)-bis (7,7-dimethyl-2-oxobicyclo-[2.2.1]heptan-1-methansulfonsäure) and salts (Mexoryl SX)
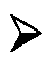 3-(4'-Sulfo)-benzyliden-bornan-2-on and salts (Mexoryl SL)
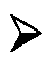 Polymer von N-{(2und 4)-[2-oxoborn-3-yliden)methyl}benzyl]acrylamid (Mexoryl SW)
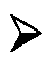 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl SL)
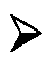 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
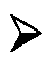 Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene);
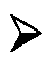 Ester der Salicylsäure, vorzugsweise Salicylsäure-2-ethylhexylester, Salicylsäure-4-iso-propylbenzylester, Salicylsäurehomomenthylester;
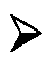 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
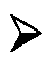 Ester der Benzalmalonsäure, vorzugsweise 4-Methoxybenzmalonsäuredi-2-ethylhexyl-ester;
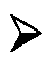 Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl)anilino]-1,3,5-triazin (Uvinul T 150) wie in der EP 0818450 A1 beschrieben oder 4,4'-[(6-[4-((1,1-Dimethylethyl)aminocarbonyl)phenyl-amino]-l,3,5-triazin-2,4-diyl)diimino] bis(benzoesäure-2-ethylhexylester) (Uvasorb® HEB);
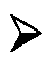 2,2(-Methylen-bis(6-(2H-benzotriazol-2-yl)-4- (1,1,3,3-tetramethyl-butyl)phenol) (Tinosorb M);
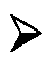 2,4-Bis[4-(2-ethylhexyloxy)-2-hydroxyphenyl]-6-(4-methoxyphenyl)-1,3,5-triazin (Tinosorb S);
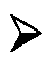 Propan-1,3-dione, wie z.B. 1-(4-tert.Butylphenyl)-3-(4'methoxyphenyl)propan-1,3-dion;
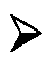 Ketotricyclo(5.2.1.0)decan-Derivate, wie in der EP 0694521 B1 beschrieben;
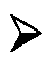 Dimethicodiethylbenzalmalonate (Parsol SLX).

Als wasserlösliche UV - Filter kommen in Frage:
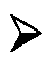 2-Phenylbenzimidazol-5-sulfonsäure und deren Alkali-, Erdalkali-, Ammonium-, Alkylammonium-, Alkanolammonium- und Glucammoniumsalze;
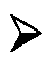 2,2(-(1,4-Phenylen)bis(1H-benzimidazol-4,6-disulfon-säure, Mononatriumsalz) (Neo Heliopan AP)
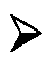 Sulfonsäurederivate von Benzophenonen, vorzugsweise 2-Hydroxy-4-methoxybenzo-phenon-5-sulfonsäure und ihre Salze;
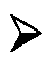 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der Fassung **Commission Directive 2005/9/EC of 28 January 2005 amending Council Directive 76/768/EEC,** concerning cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pig-mente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 8/1996, S. 543-548 sowie Parf.Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnS04) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Eine bevorzugte Ausführungsform der Erfindung betrifft kosmetischen und/oder pharmazeutische Zubereitungen, enthaltend **0,1 bis 80 Gew.% Kohlenwasserstoffe,** ausgewählt aus der Gruppe anspruchsgemäßer Kohlenwasserstoff(gemisch)e und mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden- bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)-[2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy)disiloxanyl)propyl) phenol, Dimethicodiethyl-benzalmalonate und ihren Mischungen.

Diese UV-Lichtschutzfilter sind beispielsweise unter den folgenden Handelsnamen kommerziell erhältlich:
NeoHeliopan®MBC (INCI: 4-Methylbenzylidene Camphor; Hersteller: Symrise); NeoHeliopan® BB (INCI: Benzophenone-3, Hersteller: Symrise); Parsol®1789 (INCI: Butyl Methoxydibenzoylmethane, Hersteller: Hoffmann-La Roche (Givaudan); Tinosorb®S (INCI: Bis-Ethylhexyloxyphenol Methoxyphenyl Triazine); Tinosorb®M (INCI: Methylene Bis-Benzotriazolyl Tetramethylbutylphenol): Herstelller: Ciba Specialty Chemicals Corporation; Uvasorb®HEB (INCI: Diethylhexyl Butamido Triazone, Hersteller: 3V Inc.), Uvinul®T 150 (INCI: Ethylhexyl Triazone, Hersteller: BASF AG); Uvinul® A plus (INCI: Diethylamino Hydroxybenzoyl Hexyl Benzoate: Hersteller: BASF AG; Mexoryl® SO: 3-(4'-Trimethylammonium) benzyliden- bornan-2-on-methylsulfat, INCI: Camphor Benzalkonium Methosulfate; Mexoryl®SX: 3,3'-(1,4-Phenylendimethin)-bis (7,7- dimethyl-2-oxobicyclo-[2.2.1] heptan-1-methansulfonsäure), CTFA: INCI Terephthalylidene Dicamphor Sulfonic Acid; Mexory® SL: 3-(4'-Sulfo)-benzyliden-bornan-2-on, INCI Benzylidene Camphor Sulfonic Acid; Mexoryl®SW: Polymer von N-{(2und 4)-[2-oxoborn-3-yliden)methyl}benzyl]acrylamid, INCI Polyacrylamidomethyl Benzylidene Camphor; Mexoryl®SL: 2-(2H-Benzotriazol-2-yl)-4-methyl-6-(2-methyl-3-(1,3,3,3-tetramethyl-1-(trimethylsilyloxy)disiloxanyl)propyl) phenol; INCI: DROMETRIZOLE TRISILOXANE; Parsol® SLX: Dimethicodiethylbenzalmalonate, INCI Polysilicone-15.

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf das Gesamtgewicht der kosmetischen und/oder pharmazeutischen Zubereitung - enthalten.

Ein Gegenstand der Erfindung betrifft kosmetische und/oder pharmazeutische Zubereitungen, enthaltend **0,1 bis 80 Gew.% Kohlenwasserstoffe,** ausgewählt aus der Gruppe anspruchsgemäßer Kohlenwasserstoff(gemisch)e und mindestens einen **Selbstbräuner.**

Als Selbstbräuner sind Substanzen zu verstehen, welche eine Bräunung der Haut verursachen. Beispielsweise seien Dihydroxyaceton, Erythrulose sowie alpha, betaungestättige Aldehyde genannt, welche mit den Aminosäuren der Haut im Sinne einer Maillard Reaktion zu gefärbten Verbindungen abreagieren. Als Wirkstoffe für Selbstbräuner kommen weiterhin in Frage natürliche oder synthetische Ketole oder Aldole. Als geeignete Wirkstoffe seien exemplarisch genannt Dihydroxyaceton, Erythrulose, Glycerolaldehyd, Alloxan, Hydroxymethylglyoxal, gamma-Dialdehyd, 6-Aldo-D-Fructose, Ninhydrin und meso-Weinsäuredialdehyd. **Als Selbstbräuner eignen sich insbesondere** Dihydroxyaceton und/oder Erythrulose.

Als besonders vorteilhaft haben sich Mischungen der o. g. Wirkstoffe untereinander oder mit Mucondialdehyd oder/und Naphthochinone wie z. B. 5-Hydroxy-1,4-naphthochinon (Juglon) und 2-Hydroxy-1,4-Naphthochinon erwiesen.

Die erfindungsgemäßen Zubereitungen enthalten die Selbstbräuner üblicherweise in Konzentrationen von 1 bis 10, insbesondere von 2 bis 5 Gew.-% -bezogen auf das Gesamtgewicht der kosmetischen und/oder pharmazeutischen Zubereitung.

Eine besonders bevorzugte Ausführungsform der Erfindung betrifft kosmetische und/oder pharmazeutische Zubereitungen, enthaltend **0,1 bis 80 Gew.% Kohlenwasserstoffe,** ausgewählt aus der Gruppe anspruchsgemäßer Kohlenwasserstoff(gemisch)e und mindestens einen **UV-Lichtschutzfilter** und mindestens einen **Selbstbräuner.**

Die erfindungsgemäßen kosmetischen und/oder pharmazeutischen Zubereitungen können beispielsweise als O/W oder W/O Pflegeemulsionen, Sonnenschutzformulierung, AP/Deo Konzepte, Formulierungen für die dekorative Kosmetik, ölige Pflegezubereitungen, Tränkflüssigkeiten für Substrate, wie beispielsweise Papier- und Vliessprodukte vorliegen. Exemplarisch seien genannt Wet Wipes, Taschentücher, Windeln oder Hygieneprodukte.

Die erfindungsgemäßen kosmetischen und/oder pharmazeutischen Zubereitungen, eignen sich insbesondere auch für leichte, sprühbare Anwendungen und/oder als Bestandteile von Pflegeemulsionen für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung. Sie lassen sich auf Tissues, Papiere, Wipes, Vlies-Produkte, Schwämme, Puffs, Pflaster und Bandagen applizieren, die ihren Einsatz im Bereich der Reinigung, Hygiene und/oder Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-SunTreatment, Toiletten-Feuchttücher, Antitranspirant-Wipes, Windeln, Taschentücher, Wet Wipes, Hygieneprodukte, Selbstbräunungs Wipes, Toilettenpapier, Erfrischungstücher, After-shave Tücher). Sie lassen sich u.a. auch in Zubereitungen zur Haarpflege, Haarreinigung oder Haarfärbung einsetzen. Die erfindungsgemäßen kosmetischen und/oder pharmazeutischen Zubereitungen zeichnen sich durch positives sensorisches Verhalten bei Applikation aus.

Die erfindungsgemäßen kosmetischen und/oder pharmazeutischen Zubereitungen eignen sich als Zubereitungen der dekorativen Kosmetik, wie beispielsweise Lippenstifte, Augen-Make up, wie beispielsweise Lidschatten, Mascara, Lidstifte, Kajal, Nagellack, etc. sowie Make-up Formulierungen.

Ein Gegenstand der Erfindung betrifft daher kosmetische und/oder pharmazeutische Zubereitungen, enthaltend **0,1 bis 80 Gew.-% Kohlenwasserstoffe,** ausgewählt aus der Gruppe anspruchsgemäßer Kohlenwasserstoff(gemisch)e und mindestens ein **Pigment und/oder einen Farbstoff.**

Der Begriff **Pigment** umfasst Partikel jeder Form, die weiß oder gefärbt, organisch oder anorganisch sind, in den Zubereitungen nicht löslich sind, und dem Zweck dienen die Zubereitung zu färben. In einer bevorzugten Ausführungsform werden anorganische Pigmente verwendet, besonders bevorzugt sind Metalloxide.

Als **anorganische Pigmente** seien exemplarisch genannt: Titandioxid, optional oberflächenbeschichtet, Zirkonium oder Ceriumoxide und Zink-, Eisen- (Schwarz, Gelb oder Rot) und Chrom-oxide, Manganviolett, Ultramarine Blau, Chromhydroate und Eisen(III)blau, Metallpulver wie Aluminiumpulver oder Kupferpulver.

In einer bevorzugten Ausführungsform der Erfindung ist das Pigment ausgewählt aus den anorganischen Pigmenten, vorzugweise aus den Metalloxiden. In einer bevorzugten Ausführungsform ist das Pigment ausgewählt aus der Gruppe bestehend aus Titandioxid, Zinkoxid, Eisenoxid und Mischungen daraus.

Die Pigmente können sowohl einzeln als auch in Mischungen vorliegen.

Bevorzugt im Sinne der vorliegenden Erfindung sind Pigmentmischungen aus Weiss-Pigmenten (z. B. Kaolin, Titandioxid oder Zinkoxid) und anorganischen Farbpigmenten (z. B. Eisenoxid Pigmente, Chromoxide), wobei die Pigmente beschichtet ("gecoatet") oder unbeschichtet vorliegen koennen. Unter den Farbpigmenten sind Eisenoxide besonders bevorzugt.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäss vorteilhaft zusätzlich zu einem oder mehreren Weiss- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenfoermiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenfoermige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phaenomen der Interferenz (Interferenzpigmente) (DIN 55944: 2003-11).

Beispiele für erfindungsgemäss bevorzugte handelsübliche Effektpigmente sind: Timiron and #174; von Merck, Iriodin and #174; von Merck (Perl- und Farbglanzpigmente für dekorative technische Anwendungen), Xirallic and #174; von Merck (farbintensive Kristalleffektpigmente).

Ferner können die erfindungsgemässen Zubereitungen vorteilhaft auch **organische Farbpigmente** enthalten, d. h. organische Farbstoffe, welche in der Zubereitung praktisch unlöslich sind. Nach **DIN 55944: 1990-04** können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weisspigmente sind ohne praktische Bedeutung.

Die Pigmente können vorteilhaft im Sinne der vorliegenden Erfindung auch in Form kommerziell erhältlicher öliger oder wässriger Vordispersionen zur Anwendung kommen. Die erfindungsgemäßen Zubereitungen enthalten üblicherweise 0,1 bis 40 Gew.-% Pigmente - bezogen auf das Gesamtgewicht der kosmetischen und/oder pharmazeutischen Zubereitung.

Es ist ferner vorteilhaft im Sinne der vorliegenden Erfindung, wenn die erfindungsgemässe Zubereitung einen oder mehrere **Farbstoffe** enthält.

Die Farbstoffe können sowohl synthetischen als auch natürlichen Ursprungs sein. Eine Liste von geeigneten Farbstoffen findet sich in EP 1 371 359 A2**,** S.8, Z. 25-57, S.9 und S.10 sowie S.11, Z. 1 bis 54, auf welche hiermit explizit Bezug genommen wird.

Die erfindungsgemäßen Zubereitungen enthalten üblicherweise 0,01 bis 5, vorzugsweise 0,1 bis 1,0 Gew.-% Farbstoffe- bezogen auf das Gesamtgewicht der kosmetischen und/oder pharmazeutischen Zubereitung. Die erfindungsgemäßen Zubereitungen enthalten üblicherweise eine Gesamtmenge an Farbstoffen und Pigmenten im Bereich von 0,01 bis 30 Gew.-%, insbesonderen 0,1 bis 15 Gew.-%, vorzugsweise 1 bis 10 Gew.-%, bezogen auf das Gesamtgewicht der kosmetischen und/oder pharmazeutischen Zubereitung.

Als Farbstoffe und Pigmente eignen sich insbesondere die gemäß Annex IV der Kommissions Direktive zugelassenen Farbstoffe und Pigmente (in der Fassung: **Commission Directive 2007/22/EC of 17 April 2007 amending Council Directive 76/768/EEC,** concerning cosmetic products, for the purposes of adapting Annexes IV and VI thereto to technical progress) zugelassen Stoffe, auf die hiermit explizit Bezug genommen wird.

Die kosmetischen und/oder pharmazeutischen Zubereitungen können Formulierungen zur Körperpflege sein, z. B. eine Körpermilch, Cremes, Lotionen, sprühbare Emulsionen, Produkte zur Eliminierung des Körpergeruchs etc. Die Kohlenwasserstoff Gemische lassen sich auch in tensidhaltigen Formulierungen wie z. B. Schaum- und Duschbädern, Haarshampoos und Pflegespülungen einsetzen. Je nach Applikationszweck enthalten die kosmetischen und/oder pharmazeutischen Zubereitungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Ein weiterer Gegenstand der Erfindung betrifft kosmetische und/oder pharmazeutische Zubereitungen, enthaltend **0,1 bis 80 Gew.-% Kohlenwasserstoffe** ausgewählt aus der Gruppe anspruchsgemäßer Kohlenwasserstoff(gemisch)e und mindestens einen Emulgator und/oder ein Tensid und/oder eine Wachskomponente und/oder ein Polymer und/oder einen weiteren Ölkörper.

### Emulgator

In einer Ausführungsform der Erfindung enthalten die erfindungsgemäßen Zubereitungen mindestens einen Emulgator.

Ein weiterer Gegenstand der Erfindung betrifft daher kosmetische und/oder pharmazeutische Zubereitungen, enthaltend **0,1 bis 80 Gew.-% Kohlenwasserstoffe,** ausgewählt aus der Gruppe bestehend aus C7, C9, C11, C13, C15, C17, C19, C21 und C23 Kohlenwasserstoffen und mindestens einen Emulgator.

Die erfindungsgemäßen Zubereitungen enthalten den/die Emulgator(en) üblicherweise in einer Menge von 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zubereitung.

Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Grösse und Stärke der hydrophilen und der lipophilen Gruppen eines Emulgators aus. Der HLB-Wert eines Emulgators lässt sich auch aus Inkrementen errechnen, wobei die HLB-Inkremente für die verschiedenen hydrophilen und hydrophoben Gruppen, aus denen sich ein Molekül zusammensetzt. In der Regel kann er Tabellenwerken (z. B. H. P. Fiedler, Lexikon der Hilfsstoffe für Pharmazie, Kosmetik und angrenzende Gebiete, Editio Cantor Verlag, Aulendorf, 4. Aufl. 1996) oder den Herstellerangaben entnommen werden. Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Öiphase entsteht unter sonst gleichen Herstellungsbedingungen eine W/O-Emulsion.

In einer Ausführungsform der Erfindung enthält die erfindungsgemäße Zubereitung mehr als einen Emulgator. Der Fachmann setzt in Abhängigkeit der übrigen Komponenten übliche Emulgator Systeme (wie z.B. Emulgator und Co-Emulgator) ein.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls® PGPH" (W/O-Emulgator) oder "Eumulgin®VL 75" (Abmischung mit Lauryl Glucosides im Gewichtsverhältnis 1:1, O/W-Emulgator) oder Dehymuls® SBL (W/O-Emulgator) von der Cognis Deutschland GmbH vertrieben werden. In diesem Zusammenhang sei insbesondere auf das Europäische Patent EP 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Besonders bevorzugte Emulgatoren sind z. B. Cetyl Dimethicone Copolyol (z.B. Abil EM-90), Polyglyceryl-2 Dipolyhydroxystearate (z.B. Dehymuls PGPH), Polyglycerin-3-Diisostearate (z.B. Lameform TGI), Polyglyceryl-4 Isostearate (z.B. Isolan GI 34), Polyglyceryl-3 Oleate (z.B. Isolan GO 33), Diisostearoyl Polyglyceryl-3 Diisostearate (z.B. Isolan PDI), Polyglyceryl-3 Methylglucose Distearate (z.B. Tego Care 450), Polyglyceryl-3 Beeswax (z.B. Cera Bellina), Polyglyceryl-4 Caprate (z.B. Polyglycerol Caprate T2010/90), Polyglyceryl-3 Cetyl Ether (z.B. Chimexane NL), Polyglyceryl-3 Distearate (z. B. Cremophor GS 32) und Polyglyceryl Polyricinoleate (z.B. Admul WOL 1403), Glyceryl Oleate (z.B. Monomuls 90-0 18), Alkyl Glucoside (z.B. Plantacare 1200, Emulgade PL 68/50, Montanov 68, Tego Care CG 90, Tego Glucosid L 55), Methyl Glucose Isostearate (z.B. Tego Care IS), Methyl Glucose Sesquistearate (Tego Care PS), Sodium Cocoyl Hydrolyzed Wheat Protein (z.B. Gluadin WK), Potassium Cetyl Phosphate (z.B. Amphisol K, Crodafos CKP), Sodium Alkylsulfate (z.B. Lanette E), Sucrose Ester (z.B. Crodesta F-10, F-20, F-50, F-70, F-110, F-160, SL-40, Emulgade® Sucro), ethoxylierte und/oder propoxylierte Fettalkohole Fettsäuren, Rizinusöle bzw. hydrierte Rizinusöle (z.B. Eumulgin B2, B2, B3, L, HRE 40, HRE 60, RO 40, Cremophor HRE 40, HRE 60, L, WO 7, Dehymuls HRE 7, Arlacel 989), PEG-30 Dipolyhydroxystearate (z.B. Arlacel P 135, Dehymuls LE), Sorbitan Ester, Sorbitan Ester ethoxyliert und/oder propoxyliert sowie deren Gemische. Ein besonders effektives Gemisch besteht aus Polyglyceryl-2 Dipolyhydroxystearate und Lauryl Glucoside und Glycerin (z.B. Eumulgin VL 75). Geeignet sind weiterhin Polyglyceryl-4 Diisostearate/ Polyhydroxystearate/Sebacate (Isolan® GPS), Diisostearoyl Polyglyceryl-3 Diisostearate (z. B. Isolan PDI), Alkalisalze Acylglutamate (z.B. Eumulgin SG).

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer,"Encyclopedia of Chemical Technology", 3. Aufl., 1979, Band 8, Seite 913**,** aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestem, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitan-dihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin® HRE 40 (INCI: PEG-40 Hydrogenated Castor Oil), Eumulgin® HRE 60 (INCI: PEG-60 Hydrogenated Castor Oil), Eumulgin® L (INCI: PPG-1-PEG-9 Laurylglycolether), sowie Eumulgin® SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare® zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade® PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin® VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur: 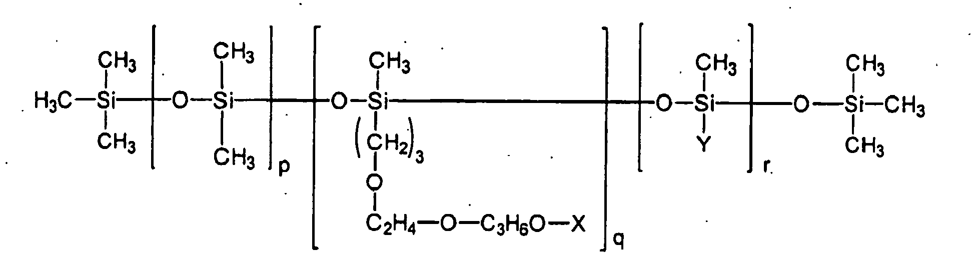 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden. Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikGoldschmidt unter der Warenbezeichnung ABIL® EM 90 verkauft wird. Ein weiteres Beispiel für besonders vorteilhaft im Sinne der vorliegenden Erfindung zu verwendende grenzflächenaktiven Substanzen ist das Cyclomethicon Dimethiconcopolyol, welches von Evonik Goldschmidt unter der Warenbezeichnung ABIL®EM 97 und ABIL®WE 09 verkauft wird. Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Corning Ltd. erhältlich ist. Ein weiterer vorteilhafter Silikonemulgator ist Octyl Dimethicon Ethoxy Glucosid der Firma Wacker.

Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z. B. ABIL® EM 90 Evonik Goldschmidt), DC5200 Formulation Aid (Dow Corning)] sowie beliebige Mischungen aus beiden Emulgatoren.

### Tenside

In einer Ausführungsform der Erfindung enthalten die erfindungsgemäßen Zubereitungen mindestens ein Tensid.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekuelteil, für eine Herabsetzung der Oberflächenspannung des Wassers, die Benetzung der Haut, die Erleichterung der Schmutzentfernung und -lösung, ein leichtes Abspülen und - je nach Wunsch- für Schaumregulierung.

Als Tenside werden üblicherweise oberflächen aktive Substanzen verstanden, die einen HLB-Wert von größer 20 aufweisen.

Ein weiterer Gegenstand der Erfindung betrifft daher kosmetische und/oder pharmazeutische Zubereitungen, enthaltend **0,1 bis 80 Gew.-% Kohlenwasserstoffe,** ausgewählt aus der Gruppe anspruchsgemäßer Kohlenwasserstoff(gemisch)e und mindestens **ein Tensid.**

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Die erfindungsgemäßen Zubereitungen enthalten den/die Tenside üblicherweise in einer Menge von 0 bis 40 Gew.-%., vorzugsweise 0,05 bis 30 Gew.-% insbesondere 0,05 bis 20 Gew.-%, vorzugsweise 0,1 bis 15 Gew.-% und insbesondere 0,1 bis 10 Gew.-% bezogen auf das Gesamtgewicht der Zubereitung.

Typische Beispiele für **nichtionische Tenside** sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als **zwitterionische Tenside** werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind **ampholytische Tenside.** Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

**Anionische Tenside** sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid-(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

### Wachskomponente

In einer Ausführungsform der Erfindung enthalten die erfindungsgemäßen Zubereitungen mindestens eine Wachskomponente.

Ein weiterer Gegenstand der Erfindung betrifft daher kosmetische und/oder pharmazeutische Zubereitungen, enthaltend **0,1 bis 80 Gew.-% Kohlenwasserstoffe,** ausgewählt aus der Gruppe anspruchsgemäßer Kohlenwasserstoff(gemisch)e und mindestens **eine Wachskomponente.**

Die erfindungsgemäßen Zubereitungen enthalten den/die Wachskomponente(n) üblicherweise in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zubereitung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten Triglycerid-Gemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden), sowie Cutina MD oder Cutina GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der Cognis Deutschland GmbH unter der Bezeichnung Lanette 18 (C18-Alkohol), Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zubereitungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat, - myristat oder -stearat kommen hierfür in Frage.

Als Wachse eignen sich weiterhin **Perlglanzwachse.** Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Polymere

In einer Ausführungsform der Erfindung enthalten die erfindungsgemäßen Zubereitungen mindestens ein Polymer.

Ein weiterer Gegenstand der Erfindung betrifft daher kosmetische und/oder pharmazeutische Zubereitungen, enthaltend **0,1 bis 80 Gew.-% Kohlenwasserstoffe,** ausgewählt aus der Gruppe anspruchsgemäßer Kohlenwasserstoff(gemisch)e und mindestens **ein Polymer.**

Die erfindungsgemäßen Zubereitungen enthalten das/die Polymere(n) üblicherweise in einer Menge von 0 bis 20 Gew.-%, vorzugsweise 0,1 bis 15 Gew.-% und insbesondere 0,1 bis 10 Gew.-% bezogen auf das Gesamtgewicht der Zubereitung.

Geeignete **kationische Polymere** sind beispielsweise kationische Cellulosederivate, wie z. B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen sowie beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox).

Ebenso geeignet sind sogenannte **quaternäre Polymere,** z.B. mit der INCI - Bezeichnung Polyquaternium-37, die der folgenden allgemeinen Formel entsprechen:
Alternativ können auch andere Dialkylaminoalkyl (meth)acrylate sowie deren durch Alkylierung oder Protonierung erhältliche Ammoniumsalze oder Dialkylaminoalkyl (meth)acrylamide sowie deren durch Alkylierung oder Protonierung erhältliche Ammoniumsalze eingesetzt werden. Besonders bevorzugt sind Polymere enthaltend MAPTAC, APTAC, MADAME, ADAME, 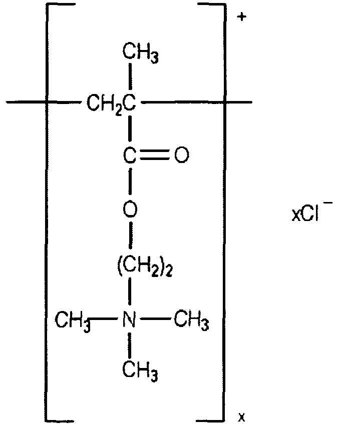 DMAEMA und TMAEMAC. Darüberhinaus können auch Co-polymere mit anionischen, weiteren kationischen oder ungeladenen Monomeren erfindungsgemäß eingesetzt werden, insbesondere solche, die neben den genannten Alkylaminoalkyl (meth)acrylat oder - (meth)acrylamid Monomeren zusätzlich (Meth)acrylsäure und/oder 2-Acrylamido-2-methyl-propansulfonsäure und/oder Acrylamid und/oder Vinylpyrrolidon und/oder Alkyl(meth)acrylate enthalten. Beispielhaft seien solche Polymere mit der INCI Bezeichnung Polyquaternium-11, Polyquaternium-13, Polyquaternium-14, Polyquaternium-15, Polyquaternium-28, Polyquaternium-32, Polyquaternium-43, Polyquaternium-47 genannt.

### Ölkörper

In einer Ausführungsform der Erfindung enthalten die erfindungsgemäßen Zubereitungen mindestens einen Ölkörper. Üblicherweise enthalten die erfindungsgemäßen Zubereitungen den oder die Kohlenwasserstoffe als Ölkörper. In der hier als bevorzugte genannten Ausführungsform enthalten die Zubereitungen somit einen von dem erfindungsgemäßen Kohlenstoffen verschiedenen Ölkörper, auch als "weiterer Ölkörper" bezeichnet.

Ein weiterer Gegenstand der Erfindung betrifft daher kosmetische und/oder pharmazeutische Zubereitungen, enthaltend **1 bis 80 Gew.-% Kohlenwasserstoffe,** ausgewählt aus der Gruppe anspruchsgemäßer Kohlenwasserstoff(gemisch)e und mindestens **einen (weiteren) Ölkörper.**

Die Ölkörper (erfindungsgemäße Kohlenwasserstoffe plus weitere Ölkörper) sind üblicherweise in einer Gesamtmenge von 0,1 - 90, insbesondere 0,1 - 80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 - 15 Gew.-% enthalten. Die weiteren Ölkörper sind üblicherweise in einer Menge von 0,1 bis 40 Gew.-% bezogen auf das Gesamtgewicht der Zubereitung enthalten.

Als weitere Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen..

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 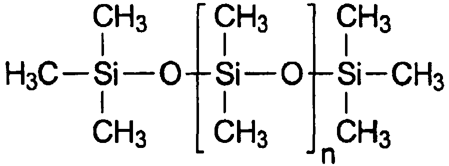 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handelsbezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclo- trisiloxan, Polydimethylsiloxan, Poly (methylphenylsiloxan). Erfindungsgemäß besonders bevorzugte Silikone sind Dimethicon und Cyclomethicon.

Die erfindungsgemäßen Zubereitungen können weiterhin biogenen Wirkstoffe, Insekten-Repellentien, Tyrosinase Inhibitoren, Konservierungsmittel, Parfümöle, Überfettungsmittel, Stabilitsatoren und/oder Hydrotrope enthalten.

Ein weiterer Gegenstand der Erfindung betrifft daher kosmetische und/oder pharmazeutische Zubereitungen, enthaltend **0,1 bis 80 Gew.-% Kohlenwasserstoffe,** ausgewählt aus der Gruppe anspruchsgemäßer Kohlenwasserstoff(gemisch)e und **mindestens einen biogenen Wirkstoff, Insekten-Repellent, Tyrosinase Inhibitor, Konservierungsmittel, Parfümöl, Stabilisator und/oder Hydrotrop.**

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Aloe vera, Prunusextrakt, Bambaranussextrakt und Vitaminkomplexe zu verstehen.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als **Tyrosinhinbitoren,** die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe. Weiterhin eignen sich als Konservierungsmittel die in WO 07/048757 beschriebenen 1,2 Alkandiole mit 5 bis 8 C-Atomen.

Als Konservierungsmittel eignen sich insbesondere die gemäß Annex VI der Kommissions Direktive (in der Fassung Commission Directive 2007/22/EC of 17 April 2007 amending Council Directive 76/768/EEC, concerning cosmetic products, for the purposes of adapting Annexes IV and VI thereto to technical progress) zugelassen Stoffe, auf die hier explizit Bezug genommen wird.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Stabilisatoren** können Metallsalze von Fettsäuren, wie z. B. Magnesium-, Aluminium- und/oder Zinkstearat bzw. -ricinoleat eingesetzt werden.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Herstellbeispiel 1 (nicht erfindungsgemäß):

### 1a) Herstellung von Tridecan aus 1-Tetradecanol

1000 g 1-Tetradecanol (4,7 mol; Lorol C 14 der Fa. Cognis) wurden in einem rührbaren Druckbehälter mit 10 g eines Nickelkatalysators (Ni-5249 P der Fa. Engelhard; Ni-Gehalt = 63 Gew.-%) vorgelegt und auf 240 °C aufgeheizt. Anschließend wurde über einen Zeitraum von 12 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt, abgelassen und filtriert. Es ergab sich eine Auswaage von 845 g Reaktionsprodukt.

Eine GC-Analyse ergibt folgende Zusammensetzung: 89,0 % Tridecan, 2,1 % Tetradecan, 4,1 % 1-Tetradecanol, 4,2 % Dimere Reaktionsprodukte. Dieses Reaktionsprodukt wurde in einer Destillation zum reinen Tridecan fraktioniert und anschließend mit Stickstoff desodoriert. Man erhält ein farbloses, dünnflüssiges und geruchsarmes Produkt.

### 1b) Herstellung von Undecan aus 1-Dodecanol

1000 g 1-Dodecanol (5,4 mol; Lorol C 12 der Fa. Cognis) wurden in einem rührbaren Druckbehälter mit 10 g eines Nickelkatalysators (Ni-5249 P der Fa. Engelhard; Ni-Gehalt = 63 Gew%) vorgelegt und auf 240°C aufgeheizt. Anschließend wurde über einen Zeitraum von 8 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt und abgelassen und filtriert. Es ergab sich eine Auswaage von 835g Reaktionsprodukt.

Eine GC-Analyse ergibt folgende Zusammensetzung: 68,4 % Undecan, 0,6 % Dodecan, 21,7 % 1-Dodecanol, 7,2 % Dimere Reaktionsprodukte. Dieses Reaktionsprodukt wurde destilliert, um das Undecan rein zu erhalten. Dieses wurde anschließend mit Stickstoff desodoriert. Man erhält ein farbloses, dünnflüssiges und geruchsarmes Produkt.

### Herstellbeispiel 2:

### 2a) Herstellung von Pentadecan aus 1-Hexadecanol

1000 g 1-Hexadecanol (Lorol C 16 der Fa. Cognis) wurden in einem rührbaren Druckbehälter mit 10 g eines Nickelkatalysators (Ni-5249 P der Fa. Engelhard; Ni-Gehalt = 63 Gew.-%) vorgelegt und auf 240°C aufgeheizt. Anschließend wurde über einen Zeitraum von 12 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt, abgelassen und filtriert. Dieses Reaktionsprodukt wurde in einer Destillation zum reinen Pentadecan fraktioniert und anschließend mit Stickstoff desodoriert. Man erhält ein farbloses, flüssiges und geruchsarmes Produkt.

### 2b) Herstellung von Heptadecan aus 1-Octadecanol

1000 g 1-Octadecanol (Lorol C 18 der Fa. Cognis) wurden in einem rührbaren Druckbehälter mit 10 g eines Nickelkatalysators (Ni-5249 P der Fa. Engelhard; Ni-Gehalt = 63 Gew.-%) vorgelegt und auf 240°C aufgeheizt. Anschließend wurde über einen Zeitraum von 12 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt, abgelassen und filtriert. Dieses Reaktionsprodukt wurde in einer Destillation zum reinen Heptadecan fraktioniert und anschließend mit Stickstoff desodoriert. Man erhält ein farbloses, geruchsarmes Produkt, das bei 40°C flüssig ist.

## Patentansprüche

1. Kosmetische und/oder pharmazeutische Zubereitungen, enthaltend 0,1 bis 80 Gew.% eines linearen Kohlenwasserstoffs, ausgewählt aus der Gruppe bestehend aus C15, C17 und C19 Kohlenwasserstoffen oder Mischungen davon, wobei diese Kohlenwasserstoffe mehr als 95 Gew.-%, bezogen auf die Summe der in der Zubereitung enthaltenden Kohlenwasserstoffe, ausmachen.

2. Zubereitungen nach Anspruch 1, **dadurch gekennzeichnet, dass** es sich um gesättigte Kohlenwasserstoffe handelt.

3. Zubereitungen nach mindestens einem der Ansprüche 1 bis 2, **dadurch gekennzeichnet, dass** die Kohlenwasserstoffe ausgewählt sind aus der Gruppe bestehend aus n-Pentadecan, n-Heptadecan und n-Nonadecan.

4. Kosmetische und/oder pharmazeutische Zubereitung nach mindestens einem der Ansprüche 1 bis 3, enthaltend mindestens einen Antiperspirant / Desodorant Wirkstoff.

5. Kosmetische und/oder pharmazeutische Zubereitung nach mindestens einem der Ansprüche 1 bis 4, enthaltend mindestens einen UV-Lichtschutzfilter.

6. Kosmetische und/oder pharmazeutische Zubereitungen nach mindestens einem der Ansprüche 1 bis 5, enthaltend mindestens einen Selbstbräuner.

7. Kosmetische und/oder pharmazeutische Zubereitungen nach mindestens einem der Ansprüche 1 bis 6, enthaltend mindestens ein Pigment und/oder Farbstoff.

8. Kosmetische und/oder pharmazeutische Zubereitungen nach einem mindestens einem der Ansprüche 1 bis 7, enthaltend mindestens einen Emulgator und/oder ein Tensid und/oder eine Wachskomponente und/oder ein Polymer und/oder einen weiteren Ölkörper.

9. Verwendung von linearen Kohlenwasserstoffen, ausgewählt aus der Gruppe bestehend aus C15, C17 und C19 Kohlenwasserstoffen oder Mischungen davon, wobei diese Kohlenwasserstoffe mehr als 95 Gew.-%, bezogen auf die Summe der in der Mischung enthaltenen Kohlenwasserstoffe, ausmachen als Ölkörper und/oder Dispergiermittel in kosmetischen und/oder pharmazeutischen Zubereitungen.

## Claims

1. A cosmetic and/or pharmaceutical formulation comprising 0.1 to 80% by weight of a linear hydrocarbon selected from the group consisting of C15, C17 and C19 hydrocarbons or mixtures thereof, where said hydrocarbons account for more than 95% by weight, based on the sum total of the hydrocarbons comprising in the formulation.

2. The formulation according to claim 1, **characterized in that** the hydrocarbons are saturated hydrocarbons.

3. The formulation according to at least one of claims 1 to 2, **characterized in that** the hydrocarbons are selected from the group consisting of n-pentadecane, n-heptadecane and n-nonadecane.

4. The cosmetic and/or pharmaceutical formulation according to at least one of claims 1 to 3, comprising at least one antiperspirant/deodorant active ingredient.

5. The cosmetic and/or pharmaceutical formulation according to at least one of claims 1 to 4, comprising at least one UV light protection filter.

6. The cosmetic and/or pharmaceutical formulation according to at least one of claims 1 to 5, comprising at least one self-tanning agent.

7. The cosmetic and/or pharmaceutical formulation according to at least one of claims 1 to 6, comprising at least one pigment and/or dye.

8. The cosmetic and/or pharmaceutical formulation according to one at least one of claims 1 to 7, comprising at least one emulsifier and/or a surfactant and/or a wax component and/or a polymer and/or a further oil body.

9. The use of linear hydrocarbons selected from the group consisting of C15, C17 and C19 hydrocarbons or mixtures thereof, where said hydrocarbons account for more than 95% by weight, based on the sum of the hydrocarbons present in the mixture, as oil bodies and/or dispersants in cosmetic and/or pharmaceutical formulations.

## Revendications

1. Préparations cosmétiques et/ou pharmaceutiques, contenant 0,1 à 80 % en poids d'un hydrocarbure linéaire, choisi dans le groupe constitué par les hydrocarbures en C15, C17 et C19 ou leurs mélanges, ces hydrocarbures représentant plus de 95 % en poids, par rapport à la somme des hydrocarbures contenant dans la préparation.

2. Préparations selon la revendication 1, **caractérisées en ce qu'**il s'agit d'hydrocarbures saturés.

3. Préparations selon au moins l'une quelconque des revendications 1 à 2, **caractérisées en ce que** les hydrocarbures sont choisis dans le groupe constitué par le n-pentadécane, le n-heptadécane et le n-nonadécane.

4. Préparation cosmétique et/ou pharmaceutique selon au moins l'une quelconque des revendications 1 à 3, contenant au moins un agent actif antiperspirant/déodorant.

5. Préparation cosmétique et/ou pharmaceutique selon au moins l'une quelconque des revendications 1 à 4, contenant au moins un filtre de protection contre la lumière UV.

6. Préparations cosmétiques et/ou pharmaceutiques selon au moins l'une quelconque des revendications 1 à 5, contenant au moins un autobronzant.

7. Préparations cosmétiques et/ou pharmaceutiques selon au moins l'une quelconque des revendications 1 à 6, contenant au moins un pigment et/ou un colorant.

8. Préparations cosmétiques et/ou pharmaceutiques selon une au moins l'une quelconque des revendications 1 à 7, contenant au moins un émulsifiant et/ou un tensioactif et/ou un composant cireux et/ou un polymère et/ou un autre corps huileux.

9. Utilisation d'hydrocarbures linéaires, choisis dans le groupe constitué par les hydrocarbures en C15, C17 et C19 ou leurs mélanges, ces hydrocarbures représentant plus de 95 % en poids, par rapport à la somme des hydrocarbures contenus dans le mélange, en tant que corps huileux et/ou agent de dispersion dans des préparations cosmétiques et/ou pharmaceutiques.
